# EUROPEAN PATENT APPLICATION

(11) **EP 4 728 966 A1**
(43) Date of publication of application: **22.04.2026**
(21) Application number: 24825894.9
(22) Date of filing: 18.06.2024
(51) Int. Cl.: A61B 3/10

(54) **TEAR FILM PHOTOGRAPHING DEVICE**

(30) Priority: 19.06.2023 JP 2023100166
(71) Applicant: KOWA COMPANY, LTD., Aichi 460-8625 (JP)
(72) Inventor: MATSUMURA Kazunori, Chofu-shi, Tokyo 182-0021 (JP)
(74) Representative: Mathys & Squire
(86) International application number: PCT/JP2024/022046
(87) International publication number: WO 2024/262493

(57) **Abstract**

In order to provide a tear film photographing apparatus capable of acquiring a captured image with which it is possible to easily grasp which part of an eye to be examined is captured in images of interference fringes, provided are: a first optical system including an objective lens disposed to face the eye to be examined, a first illumination unit that irradiates the eye to be examined with first light through the objective lens, and a first image capturing unit that receives the first light, which has been reflected by the tear film of the eye to be examined and has passed through the objective lens, and captures the images of the interference fringes formed by the tear film; a second optical system including a second illumination unit that irradiates the eye to be examined with second light, a second light reflecting unit that is disposed on an optical path of the first optical system and selectively reflects the second light reflected by a corneal surface of the eye to be examined and passing through the objective lens, and a second image capturing unit that receives the second light via the second light reflecting unit and captures an image of an anterior eye portion of the eye to be examined, the second optical system including optical components whose positional relationship is adjusted in such a manner that the second image capturing unit has a wider range than the first image capturing unit; and an image processing unit that generates a composite image of the images of the interference fringes captured by the first optical system and the image of the anterior eye portion captured by the second optical system.

## Description

### Technical Field

The present invention relates to a tear film photographing apparatus for photographing a tear film of an eye to be examined.

### Background Art

In recent years, dry eye patients have been increasing due to factors of aging or factors of an increase of work using digital devices such as personal computers, and appropriate examinations and treatments for dry eyes have been required. Observation of a tear film of an eye to be examined is indispensable in examining dry eyes, and Patent Literature 1 is proposed as an apparatus for observing the tear film, for example.

### Citation List

### Patent Literature

Patent Literature 1: JP H10-33483 A

### Summary of Invention

### Technical Problem

Including an ophthalmic apparatus described in Patent Literature 1, it is common to focus on a part of an eye to be examined to obtain a clear interference fringe image in a case of capturing images of interference fringes of the tear film of the eye to be examined. The interference fringes are clearly captured in the image thus obtained, but there is a problem that it is difficult to intuitively perceive and easily grasp which part of the eye to be examined is captured in the obtained interference fringe image.

The present invention has been made in view of the above problem, and an object thereof is to provide a tear film photographing apparatus capable of acquiring a captured image with which it is possible to easily grasp which part of an eye to be examined is captured in an interference fringe image.

### Solution to Problem

A tear film photographing apparatus according to the present invention is a tear film photographing apparatus that captures images of interference fringes formed by a tear film of an eye to be examined, and includes: a first optical system including an objective lens disposed to face the eye to be examined, a first illumination unit that irradiates the eye to be examined with first light through the objective lens, and a first image capturing unit that receives the first light, which has been reflected by the tear film of the eye to be examined and has passed through the objective lens, and captures the images of the interference fringes formed by the tear film; a second optical system including a second illumination unit that irradiates the eye to be examined with second light, a second light reflecting unit that is disposed on an optical path of the first optical system and selectively reflects the second light reflected by a corneal surface of the eye to be examined and passing through the objective lens, and a second image capturing unit that receives the second light via the second light reflecting unit and captures an image of an anterior eye portion of the eye to be examined, the second optical system including optical components whose positional relationship is adjusted in such a manner that an image capturing range of the second image capturing unit is wider than an image capturing range of the first image capturing unit; and an image processing unit that generates a composite image of an interference fringe image captured by the first optical system and an anterior eye portion image captured by the second optical system.

In addition, in the tear film photographing apparatus according to the present invention, the second optical system may further include a filter unit configured to allow insertion and removal of an infrared light transmitting filter on an optical path between the second light reflecting unit and the second image capturing unit, the second illumination unit may irradiate the eye to be examined with the second light including infrared light, the second light reflecting unit may be a dichroic mirror that reflects the infrared light, and the second optical system may insert the infrared light transmitting filter on the optical path by the filter unit and capture the image of the anterior eye portion using the infrared light by the second image capturing unit.

In addition, in the tear film photographing apparatus according to the present invention, the second optical system may further include a filter unit configured to allow insertion and removal of a visible light transmitting filter on an optical path between the second light reflecting unit and the second image capturing unit, the second illumination unit may irradiate the eye to be examined with the second light including visible light, the second light reflecting unit may be a dichroic mirror that reflects the visible light, and the second optical system may insert the visible light transmitting filter on the optical path by the filter unit and capture the anterior eye portion image using the visible light by the second image capturing unit.

In addition, in the tear film photographing apparatus according to the present invention, the dichroic mirror may have a reflectance to the visible light lower than a reflectance to the infrared light.

In addition, in the tear film photographing apparatus according to the present invention, the second optical system may further include a filter unit configured to allow insertion and removal of a fluorescence transmitting filter on an optical path between the second light reflecting unit and the second image capturing unit, the second illumination unit may irradiate the eye to be examined with the second light including excitation light, the second light reflecting unit may be a dichroic mirror that reflects fluorescence, and the second optical system may insert the fluorescence transmitting filter on the optical path by the filter unit and capture the anterior eye portion image using the fluorescence by the second image capturing unit.

In addition, in the tear film photographing apparatus according to the present invention, the image processing unit may further measure a non-invasive tear break-up time using the interference fringe image obtained by the first optical system, and measure a fluorescein tear break-up time using the anterior eye portion image obtained by the second optical system.

In addition, in the tear film photographing apparatus according to the present invention, the second optical system may be further capable of switching between an image capturing operation of capturing the image of the anterior eye portion of the eye to be examined and an alignment operation of detecting a position of the first optical system relative to the eye to be examined, and perform binning during the alignment operation as compared with the image capturing operation.

In addition, in the tear film photographing apparatus according to the present invention, the second optical system may further include a filter unit on an optical path between the second light reflecting unit and the second image capturing unit, the second illumination unit may be configured to be capable of switching a plurality of beams of light as the second light, the filter unit may be capable of selectively switching a plurality of optical filters to transmit each of the beams of light that is the second light, and the second optical system may switch irradiation light of the second illumination unit and each of the optical filters of the filter unit in conjunction with one another.

In addition, in the tear film photographing apparatus according to the present invention, the image processing unit may further have a function of generating a composite image of the interference fringe image captured by the first optical system and an externally input anterior eye portion image input from an outside, and compare the anterior eye portion image captured by the second image capturing unit with the externally input anterior eye portion image to determine an enlargement/reduction ratio of an image when the interference fringe image is combined with the externally input anterior eye portion image.

### Advantageous Effects of Invention

One or more deficiencies are solved by the embodiments of the present application.

### Brief Description of Drawings

Fig. 1 is a system block diagram illustrating an example of a configuration of a tear film photographing apparatus 1 corresponding to at least one embodiment of the present invention.
Fig. 2 is a schematic view illustrating an example of a configuration of an optical system unit 10 in the tear film photographing apparatus 1 corresponding to at least one embodiment of the present invention.
Fig. 3 is an optical path view for describing an optical path of a first optical system and an optical path of a second optical system in the tear film photographing apparatus 1 corresponding to at least one embodiment of the present invention.
Fig. 4 is an explanatory view illustrating an outline of position correction processing in the tear film photographing apparatus 1 corresponding to at least one embodiment of the present invention.
Fig. 5 is a flowchart illustrating a flow of the position correction processing in the tear film photographing apparatus 1 corresponding to at least one embodiment of the present invention.
Fig. 6 is a flowchart illustrating a flow of image processing in the tear film photographing apparatus 1 corresponding to at least one embodiment of the present invention.
Fig. 7 is a schematic view illustrating an example of a configuration of the optical system unit 10 in the tear film photographing apparatus 1 corresponding to at least one embodiment of the present invention.
Fig. 8 is a schematic view illustrating an example of a configuration of the optical system unit 10 in the tear film photographing apparatus 1 corresponding to at least one embodiment of the present invention.
Fig. 9 is a table showing an example of a combination of an anterior eye portion illumination LED and a pre-camera filter in the tear film photographing apparatus 1 corresponding to at least one embodiment of the present invention.

### Description of Embodiments

### [First Embodiment]

Hereinafter, an example of a tear film photographing apparatus according to an embodiment of the present invention will be described with reference to the drawings. The tear film photographing apparatus according to the present example is an apparatus for photographing a tear film of an eye to be examined.

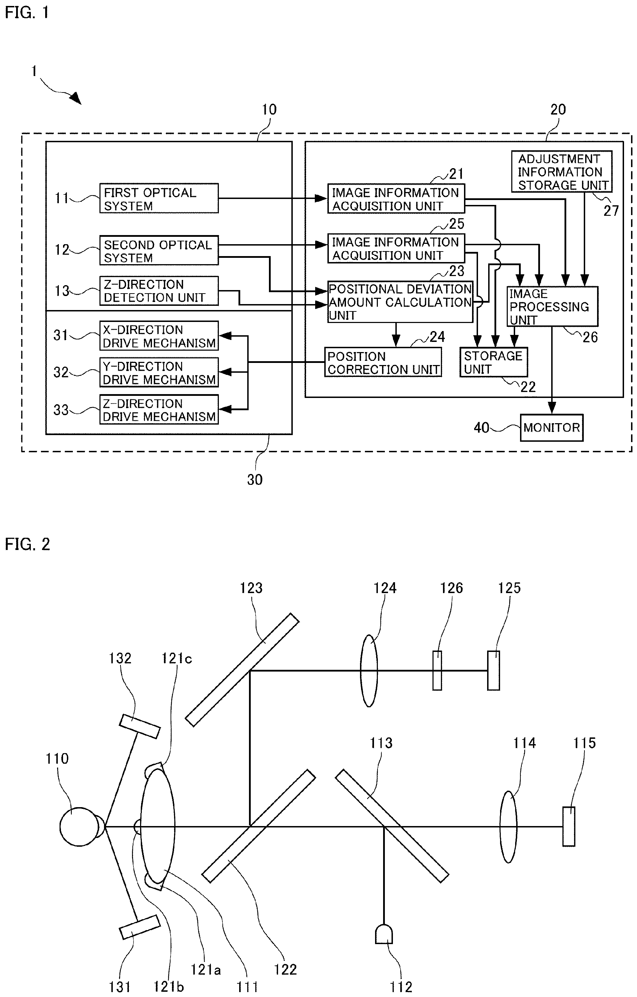

Fig. 1 is a system block diagram illustrating an example of a configuration of a tear film photographing apparatus 1 corresponding to the embodiment of the present invention. As illustrated in Fig. 1, the tear film photographing apparatus 1 includes an optical system unit 10, a control unit 20 that executes various types of arithmetic processing for implementing positional deviation detection and auto-alignment based on various types of information output from the optical system unit 10, a drive mechanism unit 30 that moves a stage supporting the optical system unit 10 based on a result of the arithmetic processing by the control unit 20, and a monitor 40 that displays a subject photographed through the optical system unit 10.

The optical system unit 10 includes a first optical system 11, a second optical system 12, and a Z-direction detection unit 13. In the embodiment of the present invention, optical members belonging to these optical systems are, for example, located on the upper side of the tear film photographing apparatus 1 and housed in an optical member housing casing (not illustrated) attached to a position facing the eye to be examined. In addition, it is preferable that the stage (not illustrated) that supports the optical members belonging to the first optical system 11, the second optical system 12, and the Z-direction detection unit 13 is housed in the optical member housing casing.

The control unit 20 includes an image information acquisition unit 21, an image information acquisition unit 25, a storage unit 22, an image processing unit 26, and an adjustment information storage unit 27. The image information acquisition unit 21 acquires captured image data of the tear film output from the first optical system 11. The image information acquisition unit 25 acquires captured image data (an image for position detection) of an anterior eye portion of the eye to be examined output from the second optical system 12. In addition, the storage unit 22 stores pieces of the captured image data acquired by the image information acquisition unit 21 and the image information acquisition unit 25. In addition, the image processing unit 26 has a function of combining pieces of the captured image data acquired by the image information acquisition unit 21 and the image information acquisition unit 25. The adjustment information storage unit 27 stores adjustment information used when the image processing unit 26 combines images. In addition, composite image data combined by the image processing unit 26 may be displayed on the monitor 40.

In addition, the control unit 20 includes a positional deviation amount calculation unit 23 and a position correction unit 24. The positional deviation amount calculation unit 23 has a function of calculating a positional deviation amount between the tear film photographing apparatus 1 and the eye to be examined based on the image for position detection output from the second optical system 12 and triangulation information output from the Z-direction detection unit 13. In addition, the positional deviation amount calculation unit 23 transmits information on the positional deviation amount to the position correction unit 24. The position correction unit 24 calculates position correction information based on the received positional deviation amount information and transmits the position correction information to the drive mechanism unit 30. In addition, the information on the positional deviation amount calculated by the positional deviation amount calculation unit 23 is also used at the time of combining images in the image processing unit 26.

Note that the control unit 20 may be a general computer including a CPU, a RAM, a hard disk, an input/output device, a communication interface, and the like. Functions achieved by the image information acquisition unit 21, the storage unit 22, the positional deviation amount calculation unit 23, the position correction unit 24, the image information acquisition unit 25, the image processing unit 26, and the adjustment information storage unit 27 described above may be implemented by a CPU or the like that executes desired arithmetic processing according to a program stored in advance. In addition, the control unit 20 may be configured to be able to communicate with an external information terminal via a communication interface.

The drive mechanism unit 30 includes an X-direction drive mechanism 31 that moves the stage supporting the optical members belonging to the first optical system 11, the second optical system 12, and the Z-direction detection unit 13 in the X direction, a Y-direction drive mechanism 32 that moves the stage in the Y direction, and a Z-direction drive mechanism 33 that moves the stage in the Z direction. Configurations of the X-direction drive mechanism 31, the Y-direction drive mechanism 32, and the Z-direction drive mechanism 33 are not particularly limited as long as the stage can be moved in the corresponding directions. For example, there is an aspect in which each of the drive mechanisms includes a motor, a gear connected to a shaft of the motor, and a rail member meshing with the gear. In this case, the motor receives the position correction information from the position correction unit 24, and rotates the shaft at a rotation speed corresponding to the received position correction information. In addition, the rail member is a member provided at a predetermined position of the stage, and converts rotational motion of the motor shaft transmitted via the gear into translational motion in each of the X, Y, and Z directions.

Fig. 2 is a schematic view illustrating an example of a configuration of the optical system unit 10 in the tear film photographing apparatus 1 corresponding to the embodiment of the present invention. In Fig. 2, the first optical system 11 includes an objective lens 111 facing an eye 110 to be examined including a tear film, a first illumination unit 112 for irradiating the eye 110 to be examined with first light through the objective lens 111, a half mirror 113, an imaging lens 114, and a first image capturing unit 115.

The first illumination unit 112 is not particularly limited as long as it is possible to illuminate the tear film of the eye 110 to be examined, but is preferably a white light source in order to allow an image of the tear film to be accurately visible through the monitor 40. Examples of the white light source include an incandescent lamp, a halogen lamp, a white LED (Light Emitting Diode) lamp, and a fluorescent lamp.

The first image capturing unit 115 is configured to receive the first light, which has been reflected by the tear film of the eye 110 to be examined and has passed through the objective lens 111, and capture images of interference fringes formed by the tear film. The first image capturing unit 115 is not particularly limited as long as it is possible to capture the image of the tear film illuminated by the first illumination unit 112. Examples of the first image capturing unit 115 include a CCD image sensor and a CMOS image sensor. Captured image data of the tear film obtained by the first image capturing unit 115 is transmitted to the control unit 20.

In the first optical system 11, illumination light from the first illumination unit 112 travels along the following optical path. That is, the illumination light (first light) emitted from the first illumination unit 112 reaches the half mirror 113, and a part thereof is reflected toward the objective lens 111. The reflected illumination light passes through the objective lens 111, then reaches the eye to be examined, is specularly reflected at the tear film of the eye to be examined, and is emitted in a direction opposite to the previous optical path. The specularly reflected illumination light passes through the objective lens 111 again and then passes through the half mirror 113. Further, the illumination light having passed through the half mirror 113 passes through the imaging lens 114 and is imaged by the first image capturing unit 115. As a result, an image of the tear film is captured.

Next, in Fig. 2, the second optical system 12 includes a second illumination unit 121 that irradiates the eye to be examined with second light, a second light reflecting unit 122 disposed on the same optical path as the first optical system 11, a mirror 123, an imaging lens 124, a second image capturing unit 125, and a filter unit 126 for blocking light having an unintended wavelength.

As illustrated in Fig. 2, the second illumination unit 121 may be a plurality of first positional deviation detection light sources 121a to 121c that are disposed around the objective lens 111 and emit the second light. Although various light sources can be employed as the first positional deviation detection light sources 121a to 121c as long as it is possible to emit the second light used for detecting the position of the optical system unit 10 with respect to the eye 110 to be examined, light sources that can be separated from the first light having a common optical axis are preferably employed, and for example, it is conceivable to employ near-infrared LEDs as light sources having different wavelengths. In addition, it is preferable to employ three first positional deviation detection light sources 121a to 121c such that the position detection can be executed by a method to be described later. An image of a state in which bright spots irradiated by the three first positional deviation detection light sources 121a to 121c are reflected on the anterior eye portion of the eye 110 to be examined is captured, and the positional deviation can be detected using information on the three bright spots appearing in the captured image.

The second light reflecting unit 122 is disposed on the same optical path as the first optical system 11, and selectively reflects the second light which has been reflected by a corneal surface of the eye to be examined and has passed through the objective lens 111. Any one that can selectively reflect the second light may be used, for example, it is conceivable to employ a dichroic mirror having a property of reflecting light having a specific wavelength and transmitting light having the other wavelengths.

The second image capturing unit 125 is configured to receive the second light via the second light reflecting unit 122 and capture an image of the anterior eye portion of the eye 110 to be examined. The second image capturing unit 125 is not particularly limited as long as it is possible to capture the image of the anterior eye portion of the eye to be examined illuminated by the second illumination unit 121. Examples of the second image capturing unit 125 include a CCD image sensor and a CMOS image sensor. Captured image data of the anterior eye portion of the eye to be examined obtained by the second image capturing unit 125 is transmitted to the control unit 20.

In the second optical system 12, illumination light from the second illumination unit 121 (the first positional deviation detection light sources 121a to 121c) travels along the following optical path. That is, the second light emitted from the second illumination unit 121 is specularly reflected by the anterior eye portion of the eye to be examined and enters the objective lens 111. The specularly reflected second light passes through the objective lens 111 and then reaches the second light reflecting unit 122. In the second light reflecting unit 122, the second light is selectively reflected, passes through the imaging lens 124 while being reflected by the mirror 123, and is imaged by the second image capturing unit 125. As a result, the anterior eye portion of the eye to be examined in the state of being irradiated with the second light is photographed.

Here, a positional relationship among the optical components is adjusted such that an image capturing range in the second image capturing unit 125 is wider than an image capturing range in the first image capturing unit 115. Therefore, since the captured image in the second image capturing unit 125 is desired to be used for positional deviation detection, a state in which a wide range of the anterior eye portion of the eye to be examined is captured is more suitable for the positional deviation detection, whereas the captured image in the first image capturing unit 115 is required to be an image in which the images of the interference fringes due to the tear film are clearly captured by enlarging a part of the eye to be examined for dry eye diagnosis. In order to achieve these two image capturing needs based on the common optical axis, the positional relationship of the optical components is adjusted such that the first image capturing unit 115 has the image capturing range in which a minute part of the anterior eye portion of the eye to be examined is enlarged and the second image capturing unit 125 has a wide range of the anterior eye portion of the eye to be examined as the image capturing range when the positional relationship is adjusted to be appropriate.

Note that the second image capturing unit 125 may be capable of switching the image quality of capturing. For example, it is conceivable that a low image quality mode with a high frame rate and low resolution (for example, the frame rate is 60 fps and the resolution is 640 × 480 pixels) is set at the time of alignment, and a high image quality mode with a low frame rate and high resolution (for example, the frame rate is 15 fps and the resolution is 2560 × 1920 pixels) is set in a stage where the alignment has converged and image capturing is executed. It can be said that the low image quality mode is advantageous in order to prioritize the processing speed and to enable alignment control to be frequently executed at the time of alignment, and the high image quality mode is advantageous in which the quality of a final composite image can also be improved by improving the image quality of an anterior eye portion image at the time of image capturing. In addition, in the low image quality mode, charges accumulated in several pixels (for example, four pixels) may be combined to form a single large pixel by performing binning may be performed when a CCD image sensor or a CMOS image sensor constituting the second image capturing unit 125 is used, thereby achieving low resolution. In the case of switching to the high image quality mode, the binning is canceled to return to high resolution.

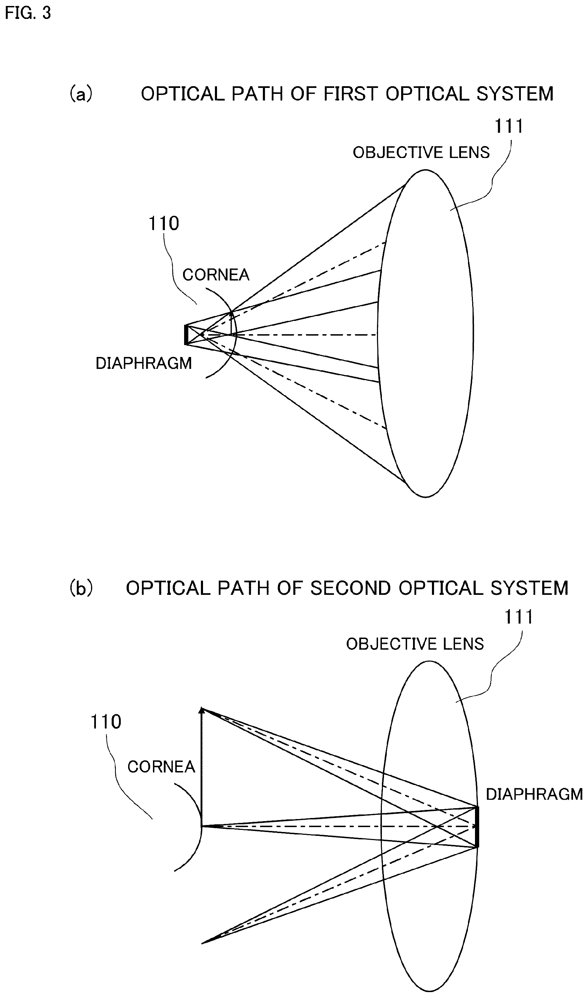

Fig. 3 is an optical path view for describing an optical path of the first optical system and an optical path of the second optical system in the tear film photographing apparatus 1 corresponding to the embodiment of the present invention. Specifically, for example, as illustrated in Fig. 3(a), in the first optical system including the first image capturing unit 115, in order to more easily obtain interference light from a cornea of the eye 110 to be examined, an exit pupil is provided near a center of a corneal curvature with an intention that a light beam is reflected in the normal direction with respect to the corneal curvature. On the other hand, as illustrated in Fig. 3(b), the second optical system including the second image capturing unit 125 is designed such that an exit pupil is provided near the objective lens 111 because the objective lens 111 becomes large when the same exit pupil position as the optical system of the first image capturing unit 115 is set in order to capture a wider range.

In addition, as illustrated in Fig. 2, the Z-direction detection unit 13 includes a second positional deviation detection light source 131 that irradiates the eye to be examined with third light, and a light receiving sensor 132 that receives the third light reflected by the anterior eye portion of the eye to be examined. The second positional deviation detection light source 131 may be any light source as long as reflection by the anterior eye portion of the eye to be examined and reception by the light receiving sensor 132 are possible, and for example, an LED, an organic light emitting diode (OLED), and various lasers are conceivable. The light receiving sensor 132 may be any sensor as long as it is possible to receive the third light reflected by the anterior eye portion of the eye to be examined, and for example, a photodiode (PD) is conceivable. In addition, the light receiving sensor 132 in the present example is preferably a split sensor capable of distinguishing two light receiving areas and performing signal conversion. As an example, it is conceivable to dispose two photodiodes side by side or employ a two-split photodiode. The second positional deviation detection light source 131 is fixed so as to be able to emit the third light at an angle at which its optical axis collides with the pupil center of the eye to be examined when the anterior eye portion of the eye to be examined exists at the ideal position, and the light receiving sensor 132 is fixed such that the third light, reflected at the pupil center of the eye to be examined when the anterior eye portion of the eye to be examined exists at the ideal position, is substantially equally applied to the two light receiving areas to obtain equal detection signals. When such positioning is performed, the detection signal of one photodiode becomes strong in a case where the eye to be examined is farther than the ideal position, and the detection signal of the other photodiode becomes strong in a case where the eye to be examined is closer than the ideal position, so that it is possible to detect the positional deviation in the Z direction.

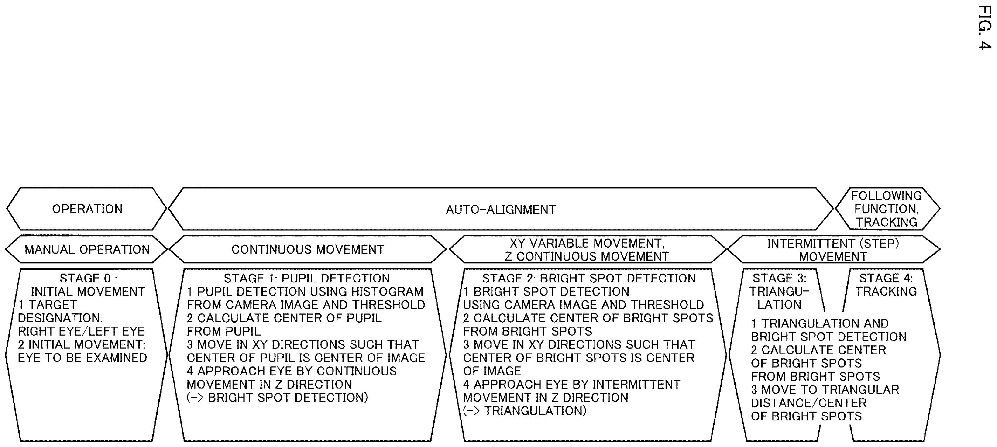

Fig. 4 is an explanatory view illustrating an outline of position correction processing in the tear film photographing apparatus 1 corresponding to the embodiment of the present invention. As illustrated in Fig. 4, the position correction in the tear film photographing apparatus 1 has three stages, that is, a stage of operation by an operator, a stage of auto-alignment, and a stage of following and tracking. First, in the stage of operation by the operator, an initial position is designated as stage 0 based on manual operation by the operator. Specifically, designation of an eye to be examined of an examinee for determining either the right eye or the left eye, and initial movement of the apparatus with respect to the eye to be examined are performed.

Next, in the stage of auto-alignment, a pupil of the eye to be examined is detected to perform coarse position correction in the X-Y direction as stage 1. First, an image for position detection obtained by capturing an image of an anterior eye portion of the eye to be examined by the second image capturing unit 125 of the second optical system 12 is acquired. Then, a position of the pupil appearing in the acquired image for position detection is detected based on image processing. Any means may be used as long as the position of the pupil can be detected. As an example of the image processing for the pupil detection, a histogram for luminance values of the image for position detection is generated, and binarization processing is performed on the image for position detection by counting the number of pixels from the low luminance side using a luminance value reaching a predetermined ratio of the number of pixels to the total number of pixels, for example, a luminance value at the time of reaching 20% of the number of pixels as a threshold. In this case, there is a high possibility of obtaining a binarized image in which a portion of the pupil is processed in black (to have a luminance of 0) and a portion around the pupil is processed in white (to have the maximum luminance). Based on this, the position of the pupil is detected, and then a center of the pupil is calculated. A positional deviation amount between the center of the pupil and a center of the current captured image is detected. Then, a position correction amount necessary for superimposing the center of the captured image on the center of the pupil is calculated. Position correction in the X-Y direction is executed by driving and controlling the X-direction drive mechanism 31 and the Y-direction drive mechanism 32 of the drive mechanism unit 30 based on the calculated position correction amount. The position correction in the X-Y direction is continued until the positional deviation amount between the position of the center of the pupil and the position of the center of the captured image becomes a predetermined target value or less.

When the positional deviation amount in the X-Y direction reaches the predetermined target value or less, coarse position correction in the Z direction is further executed as the stage 1. Specifically, movement control in the Z direction is executed in a direction approaching the eye until the light receiving sensor 132 in the Z-direction detection unit 13 detects signals. Alternatively, bright spots of Purkinje images generated in a cornea by the second light from the first positional deviation detection light sources 121a to 121c are detected, and based on a distance between the bright spots, the movement control in the Z direction is executed in the direction approaching the eye until the distance falls within a certain range in which the distance in the Z direction can be calculated. In order to further increase the accuracy, a necessary distance between bright spots may be corrected by inputting a curvature of the cornea using another apparatus. Alternatively, control is performed using these two methods in combination.

Next, as stage 2 in the stage of auto-alignment, precise position correction in the X-Y direction is performed. Bright spots of Purkinje images generated in the cornea by the second light from the first positional deviation detection light sources 121a to 121c are detected, and coordinates of a center of the bright spots are calculated using three or two bright spots. Since the center of coordinates of the three bright spots is equivalent to the center of a circle inscribed in the three bright spots, the coordinates of the center of the bright spots can be mathematically obtained from the relationship between each of the bright spots and the center. When the coordinates of the center of the bright spots are defined, a positional deviation amount from position coordinates of the center of the captured image can be calculated. A position correction amount is calculated based on the calculated positional deviation amount. Position correction in the X-Y direction is executed by driving and controlling the X-direction drive mechanism 31 and the Y-direction drive mechanism 32 of the drive mechanism unit 30 based on the calculated position correction amount. This precise position correction in the X-Y direction is continued until the positional deviation amount between the coordinates of the center of the bright spots and the position coordinates of the center of the captured image becomes a predetermined target value A or less.

When the positional deviation amount between the coordinates of the center of the bright spots and the position coordinates of the center of the captured image is the predetermined target value A or less, a target value B of the positional deviation amount less than the target value A is set in order to perform more precise control, and a more precise position correction amount is calculated so as to be the target value B or less. At this time, position correction is set to be performed with higher accuracy than that in the case of performing the position correction aiming at the target value A, for example, by finely changing a movement amount, setting a time interval for control to be short, or the like. This precise position correction in the X-Y direction is continued until the positional deviation amount between the coordinates of the center of the bright spots and the position coordinates of the center of the captured image becomes the predetermined target value B or less.

When the positional deviation amount in the X-Y direction is the predetermined target value B or less, next, precise position correction in the Z direction is performed as the stage 2 in the stage of auto-alignment. In the precise position correction in the Z direction, a positional deviation amount in the Z direction is calculated based on detection signals of the two areas of the light receiving sensor 132 that receives the third light from the second positional deviation detection light source 131 in the Z-direction detection unit 13. Specifically, since the light receiving sensor 132 is set in a state in which the third light reflected by the pupil center of the eye to be examined is substantially equally applied to the two light receiving areas and equal detection signals are obtained when the anterior eye portion of the eye to be examined is at the ideal position, a position correction amount in the Z direction is calculated from a difference between the detection signals in the two light receiving areas, and the Z-direction drive mechanism 33 is driven and controlled to execute the position correction in the Z direction. At this time, the position correction in the Z direction is executed based on a finer movement amount than that in the case of coarse position correction in the stage 1 by finely setting a control amount of the drive motor or the like. In a stage where the positional deviation amount in the Z direction becomes a predetermined target value or less, a target in the stage 2, which is the stage of auto-alignment with respect to the eye to be examined, is achieved.

Next, the stage of following and tracking is a stage for maintaining an ideal position with respect to the eye to be examined achieved by the auto-alignment. As stage 3 and stage 4, the precise position correction in the X-Y direction and the precise position correction in the Z direction up to the stage 2 are continuously executed. At this time, following and tracking may be implemented by exactly the same control up to the stage 2, or control finer than that in the stage 2 may be executed by increasing a monitoring frame rate for operation, setting a drive amount of an actuator to be operated more finely, or the like.

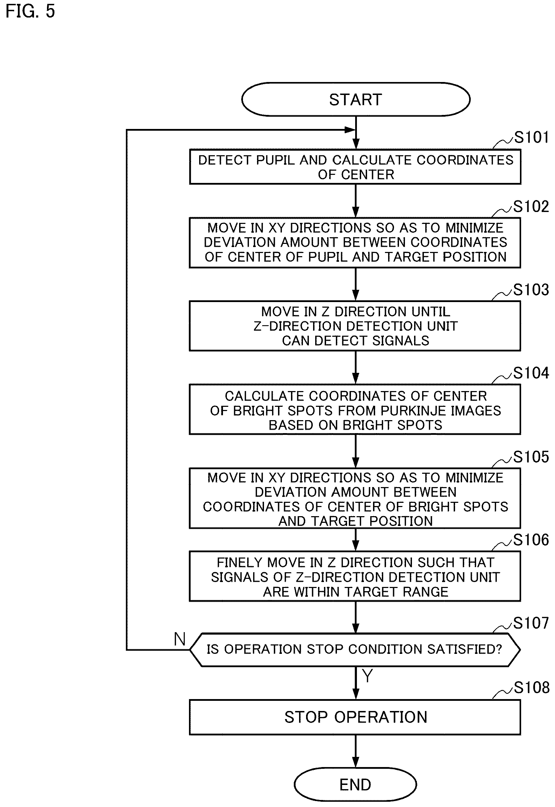

Fig. 5 is a flowchart illustrating a flow of the position correction processing in the tear film photographing apparatus 1 corresponding to the embodiment of the present invention. As illustrated in Fig. 5, the position correction processing in the tear film photographing apparatus 1 is started by detecting a pupil from an eye to be examined appearing in an acquired image for position detection and calculating coordinates of a center of the pupil (step S101). Next, the tear film photographing apparatus 1 calculates a positional deviation amount from the coordinates of the center of the pupil and target position coordinates, and moves in the X-Y direction (coarse position correction in the X-Y direction) so as to minimize the positional deviation amount (step S102). Next, the tear film photographing apparatus 1 moves in the Z direction (coarse position correction in the Z direction) until the Z-direction detection unit 13 detects signals (step S103). Next, the tear film photographing apparatus 1 calculates coordinates of a center of bright spots from Purkinje images based on the bright spots (step S104). Next, the tear film photographing apparatus 1 moves in the X-Y direction (precise position correction in the X-Y direction) so as to minimize a positional deviation amount between the coordinates of the center of the bright spots and a target position (step S105). Next, the tear film photographing apparatus 1 finely moves in the Z direction (precise position correction in the Z direction) such that the signals of the Z-direction detection unit 13 fall within a target range (step S106). Then, the tear film photographing apparatus 1 determines whether an operation stop condition is satisfied (step S107), repeatedly executes steps S101 to S106 to perform following and tracking in a case where the operation stop condition is not satisfied (S107-N), and in a case where the operation stop condition is satisfied (S107-Y), stops the operation (step S108) and then ends the position correction processing.

The present invention is characterized by performing processing of combining captured image data of a tear film such as images of interference fringes captured by the first image capturing unit 115 in the first optical system 11, the captured image data of the tear film such as the images of the interference fringes captured by the first image capturing unit 115 in the first optical system 11, and captured image data of an anterior eye portion captured by the second image capturing unit 125 in the second optical system 12.

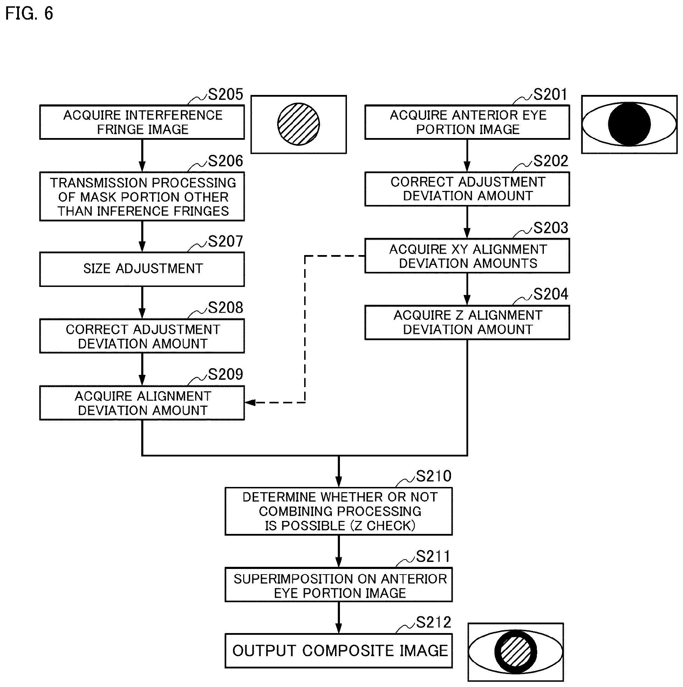

Fig. 6 is a flowchart illustrating a flow of the image processing in the tear film photographing apparatus 1 corresponding to at least one embodiment of the present invention. As illustrated in Fig. 6, the image processing executed in the image processing unit 26 of the control unit 20 is started by acquiring the captured image data (the anterior eye portion image) of the anterior eye portion captured by the second image capturing unit 125 in the second optical system 12 (step S201). Next, the image processing unit 26 acquires the adjustment information from the adjustment information storage unit 27 and corrects an adjustment deviation (step S202). Basically, the alignment is completed by aligning the center positions of the two optical systems, but it is difficult to set the deviation amount to be 0. Therefore, the deviation amount of the center position with respect to a chart at the time of adjustment for each of the anterior eye portion image and the interference fringe image is stored and held in the adjustment information storage unit 27 as the adjustment information, and the deviation is corrected using the adjustment information at the time of combining. Next, in a case where the alignment is being executed such as a case where the position of the eye is not at the center, the image processing unit 26 acquires information on an alignment deviation amount in the X-Y direction (step S203). Next, the image processing unit 26 acquires information on the alignment deviation amount in the Z direction (step S204). Since the interference fringe image cannot be acquired in the first place in a state where a deviation in the Z direction is large, the combining processing is executed only when the deviation in the Z direction falls within a certain range.

Next, the image processing unit 26 acquires captured image data (the interference fringe image) of the tear film captured by the first image capturing unit 115 in the first optical system 11 (step S205). Next, the image processing unit 26 executes transmission processing of a mask portion other than interference fringes (step S206). Next, the image processing unit 26 executes size adjustment (step S207). Next, the image processing unit 26 acquires the adjustment information from the adjustment information storage unit 27 and corrects the adjustment deviation (step S208). Next, the image processing unit 26 acquires information on the alignment deviation amount (step S209).

Next, the image processing unit 26 determines whether or not the combining processing is possible based on the positional deviation amount in the Z direction (step S210). In a case where it is determined that the combining processing is possible, the image processing unit 26 executes processing of superimposing the interference fringe image on the anterior eye portion image (step S211). Then, the image processing unit 26 outputs the obtained composite image (step S212), and ends the image processing. Note that the processing on the anterior eye portion image in steps S201 to S204 and the processing on the interference fringe image in steps S205 to S209 in Fig. 6 may be performed simultaneously in parallel. The combining processing in the image processing unit 26 of the present example can be executed on both a still image and a moving image. In a case where the combining processing is executed on each frame of a moving image, the processing on the anterior eye portion image and the processing on the interference fringe image are simultaneously performed in parallel for each frame, and results are output, thereby implementing the combining processing also on the moving image.

As described above, according to the tear film photographing apparatus of the present invention, the tear film photographing apparatus that captures images of interference fringes formed by a tear film of an eye to be examined is set to include: a first optical system including an objective lens disposed to face the eye to be examined, a first illumination unit that irradiates the eye to be examined with first light through the objective lens, and a first image capturing unit that receives the first light, which has been reflected by the tear film of the eye to be examined and has passed through the objective lens, and captures the images of the interference fringes formed by the tear film; a second optical system including a second illumination unit that irradiates the eye to be examined with second light, a second light reflecting unit that is disposed on an optical path of the first optical system and selectively reflects the second light reflected by a corneal surface of the eye to be examined and passing through the objective lens, and a second image capturing unit that receives the second light via the second light reflecting unit and captures an image of an anterior eye portion of the eye to be examined, the second optical system including optical components whose positional relationship is adjusted in such a manner that an image capturing range of the second image capturing unit is wider than an image capturing range of the first image capturing unit; and an image processing unit that generates a composite image of an interference fringe image captured by the first optical system and an anterior eye portion image captured by the second optical system. Therefore, it is possible to obtain an effect of easily grasping which portion of the eye to be examined the interference fringes are located without impairing clarity of the interference fringe image by using the composite image in which the interference fringe image is superimposed at an accurate position on the anterior eye portion image in which a wide range of the anterior eye portion of the eye to be examined is captured.

### [Second Embodiment]

In the first embodiment, it has been described that one type of light source is employed (for example, a near-infrared LED) as the second illumination unit 121 in the second optical system 12, but a plurality of types of light sources may be employed as the second illumination unit 121 to enable a plurality of types of observation and image capturing.

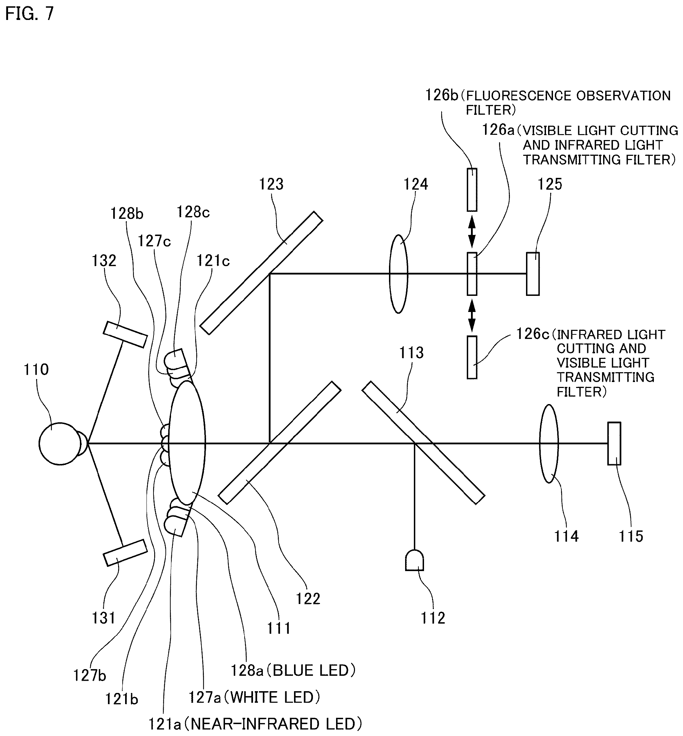

Fig. 7 is a schematic view illustrating an example of a configuration of the optical system unit 10 in the tear film photographing apparatus 1 corresponding to at least one embodiment of the present invention. In the example of Fig. 7, the second illumination unit 121 has a configuration in which the first positional deviation detection light sources 121a to 121c that emit a first type of second light, first positional deviation detection light sources 127a to 127c that emit a second type of second light, and first positional deviation detection light sources 128a to 128c that emit a third type of second light are disposed around the objective lens 111. These three types of second light can be freely switched and emitted. In addition, filter units 126a, 126b, and 126c are configured to be switchable in conjunction with switching of the light source of the second light.

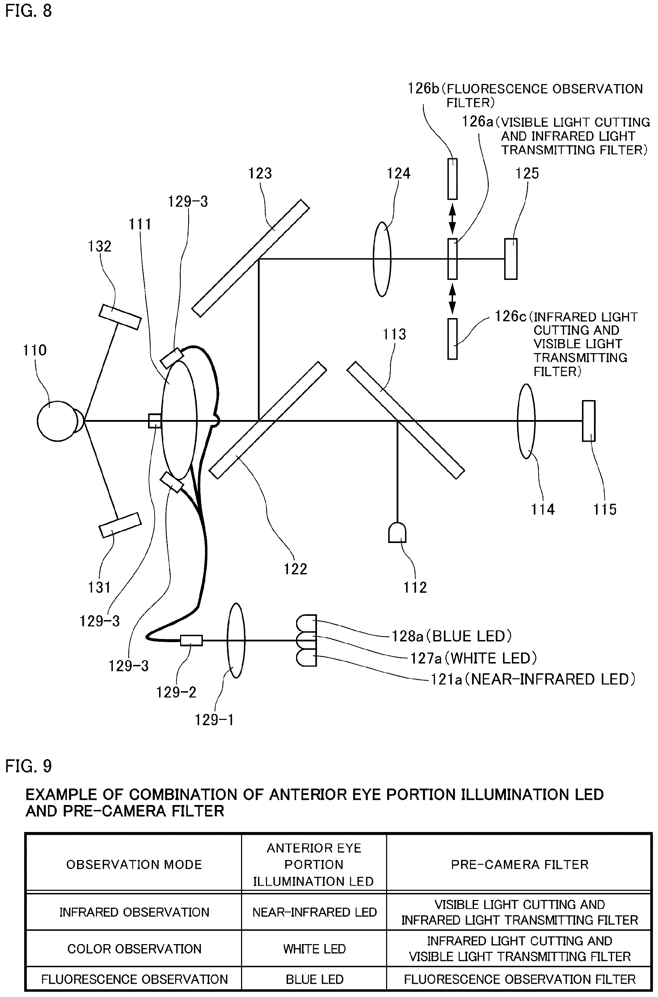

Fig. 8 is a schematic view illustrating an example of a configuration of the optical system unit 10 in the tear film photographing apparatus 1 corresponding to at least one embodiment of the present invention. The example of Fig. 8 is an example in which the light sources directly disposed around the objective lens 111 in Fig. 7 are substituted by an optical fiber cable. In the example of Fig. 8, three of the first positional deviation detection light source 121a that emits the first type of second light, the first positional deviation detection light source 127a that emits the second type of second light, and the first positional deviation detection light source 128a that emits the third type of second light are disposed side by side in a predetermined portion, and light from any one of these is condensed by a condenser lens 129-1 and made incident on an optical fiber inlet 129-2, and then caused to branch into three beams to irradiate the eye 110 to be examined from three optical fiber outlets 129-3. Similarly to the case of Fig. 7, these three types of second light can be emitted by being freely switched, and the filter units 126a, 126b, and 126c can be switched in conjunction with the switching of the light source of the second light. Note that the switching of the filter unit may be configured to be automatically switchable, or may be configured to be switched by a worker inserting and removing the filter.

Fig. 9 is a table showing an example of a combination of an anterior eye portion illumination LED and a pre-camera filter in the tear film photographing apparatus 1 corresponding to at least one embodiment of the present invention. As illustrated in Fig. 9, it is conceivable to switch three modes of infrared observation, color observation, and fluorescence observation as observation modes. In the infrared observation mode, a near-infrared LED is employed as the anterior eye portion illumination LED (the first positional deviation detection light source 121), and a visible light cutting and infrared light transmitting filter (including a case of being simply expressed as an infrared light transmitting filter) is applied as the pre-camera filter, whereby appropriate infrared observation can be performed. In the color observation mode, a white LED is employed as the anterior eye portion illumination LED (the first positional deviation detection light source 121), and an infrared light cutting and visible light transmitting filter (including a case of being simply expressed as a visible light transmitting filter) is applied as the pre-camera filter, whereby appropriate color observation can be performed. In the fluorescence observation mode, a blue LED is employed as the anterior eye portion illumination LED (the first positional deviation detection light source 121), and a fluorescence observation filter is applied as the pre-camera filter, whereby appropriate fluorescence observation can be performed.

In addition, as illustrated in Figs. 7 and 8, in the case of configuring the plurality of observation modes in a switchable manner, it is preferable to adjust a reflectance and a transmittance of light of a dichroic mirror employed as the second light reflecting unit 122. As a specific example, it is conceivable to set a reflectance of the dichroic mirror with respect to visible light to be lower than a reflectance with respect to infrared light. As described above, when the dichroic mirror that allows several% of light in a visible range to reach the second image capturing unit 125 of the second optical system 12 is employed such that both the visible light and the infrared light reach the second image capturing unit 125, both alignment processing using the infrared light and image capturing processing of an anterior eye portion using the visible light can be switched by switching the visible light cutting and infrared light transmitting filter and the infrared light cutting and visible light transmitting filter.

In addition, in the fluorescence observation mode, a non-invasive tear break-up time may be measured using an interference fringe image captured by a first image capturing unit of a first optical system by image processing in an image processing unit, and a fluorescein tear break-up time may be measured using an anterior eye portion image captured by the second image capturing unit of the second optical system. That is, since it is possible to automatically calculate FBUT (fluorescein break-up time) and NIBUT (non-invasive break-up time of a tear film) used in dry eye diagnosis, it is possible to acquire data effective as diagnosis assistance for doctors.

As described above, according to the tear film photographing apparatus of the present invention, the second optical system is set to include the filter unit configured to allow insertion and removal of an infrared light transmitting filter on an optical path between the second light reflecting unit and the second image capturing unit, the second illumination unit irradiates the eye to be examined with the second light including the infrared light, the second light reflecting unit is the dichroic mirror that reflects the infrared light, and the second optical system inserts the infrared light transmitting filter on the optical path by the filter unit and captures an image of the anterior eye portion using the infrared light by the second image capturing unit, so that the alignment processing using the infrared light can be performed.

In addition, the second optical system is set to include the filter unit capable of inserting and removing the visible light transmitting filter on the optical path between the second light reflecting unit and the second image capturing unit, the second illumination unit irradiates the eye to be examined with the second light including the visible light, the second light reflecting unit is the dichroic mirror that reflects the visible light, and the second optical system inserts the visible light transmitting filter on the optical path by the filter unit and captures the image of the anterior eye portion using the visible light by the second image capturing unit, so that the image capturing processing of the anterior eye portion using the visible light can be performed.

In addition, since the reflectance of the dichroic mirror with respect to the visible light is set to be lower than the reflectance with respect to the infrared light, it is possible to switch between the infrared observation and the color observation in the second image capturing unit by reflecting the visible light at a predetermined ratio instead of full transmission.

In addition, the second optical system is set to include the filter unit capable of inserting and removing the fluorescence transmitting filter on the optical path between the second light reflecting unit and the second image capturing unit, the second illumination unit irradiates the eye to be examined with the second light including excitation light, the second light reflecting unit is the dichroic mirror that reflects fluorescence, and the second optical system inserts the fluorescence transmitting filter on the optical path by the filter unit and captures the image of the anterior eye portion using the fluorescence by the second image capturing unit, so that the fluorescence observation using blue light can be performed.

In addition, the image processing unit is set to measure the non-invasive tear break-up time using the interference fringe image obtained by the first optical system and measure the fluorescein tear break-up time using the anterior eye portion image obtained by the second optical system, so that it is possible to acquire data effective for the dry eyes diagnosis such as FBUT and NIBUT.

In addition, the second optical system is set to be capable of switching between an image capturing operation of capturing the image of the anterior eye portion of the eye to be examined and an alignment operation of detecting a position of the first optical system relative to the eye to be examined, and perform binning during the alignment operation as compared with the image capturing operation, so that processing can be performed with low resolution during the alignment operation, and processing can be performed with high resolution during a captured moving image.

In addition, the second optical system is set to include the filter unit on the optical path between the second light reflecting unit and the second image capturing unit, the second illumination unit is capable of switching the plurality of beams of light as the second light, the filter unit is capable of selectively switching a plurality of optical filters to transmit each of the beams of light that is the second light, and the second optical system switches irradiation light of the second illumination unit and each of the optical filters of the filter unit in conjunction with one another, so that it is possible to automatically implement switching of the observation mode according to the type of the second light.

### [Third Embodiment]

In the first and second embodiments, it has been described that the interference fringe image captured by the first image capturing unit 115 of the first optical system 11 is combined with the anterior eye portion image simultaneously captured by the second image capturing unit 125 of the second optical system 12, but the purpose of image combining is to grasp which part of an eye to be examined is captured in the interference fringe image, and it is not necessarily limited to being combined with the simultaneously captured anterior eye portion image.

For example, the image processing unit 26 may have a function of generating a composite image of the interference fringe image captured by the first optical system 11 and an externally input anterior eye portion image input from the outside. That is, even if the interference fringe image is combined with the externally input anterior eye portion image captured at another timing and input to the tear film photographing apparatus 1, instead of the simultaneously captured anterior eye portion image, it is possible to grasp which part of the eye 110 to be examined is captured in the interference fringe image. In addition, by preparing the externally input anterior eye portion image having higher definition and higher image quality than the anterior eye portion image simultaneously captured by the second image capturing unit 125 of the second optical system 12, it is possible to obtain an effect of improving the image quality of the image after being combined with the interference fringe image.

In addition, in the image processing unit 26, in a case where the interference fringe image is combined with the externally input anterior eye portion image, the anterior eye portion image captured by the second image capturing unit 125 may be compared with the externally input anterior eye portion image to determine an enlargement/reduction ratio of the image at the time of combining the interference fringe image with the externally input anterior eye portion image. It is assumed that the size of the eye 110 to be examined appearing in the image is different between the anterior eye portion image captured by the second image capturing unit 125 simultaneously with the interference fringe image and the externally input anterior eye portion image. Therefore, when the enlargement/reduction ratio for the interference fringe image to be combined with the externally input anterior eye portion image is determined based on the comparison between the anterior eye portion image obtained by the second image capturing unit 125 and the externally input anterior eye portion image, it is possible to implement combining without discomfort. The enlargement/reduction ratio may be determined by any method, and for example, a method of determining the enlargement/reduction ratio by comparing the size of the iris in the anterior eye portion of the eye 110 to be examined is considered.

As described above, the image processing unit is set to have the function of generating the composite image of the interference fringe image captured by the first optical system and the externally input anterior eye portion image input from the outside, and compare the anterior eye portion image captured by the second image capturing unit with the externally input anterior eye portion image to determine the enlargement/reduction ratio of the image when the interference fringe image is combined with the externally input anterior eye portion image, so that it is possible to acquire the composite image using the externally input anterior eye portion image that is captured from the outside and has higher resolution.

### Reference Signs List

- 1: Tear film photographing apparatus
- 10: Optical system unit
- 11: First optical system
- 110: Eye to be examined
- 111: Objective lens
- 112: First illumination unit
- 113: Half mirror
- 114: Imaging lens
- 115: First image capturing unit
- 12: Second optical system
- 121: Second illumination unit
- 121a to 121c: First positional deviation detection light source
- 122: Second light reflecting unit
- 123: Mirror
- 124: Imaging lens
- 125: Second image capturing unit
- 126, 126a to 126c: Filter unit
- 127a to 127c: First positional deviation detection light source
- 128a to 128c: First positional deviation detection light source
- 129-1: Condenser lens
- 129-2: Optical fiber inlet
- 129-3: Optical fiber outlet
- 13: Z-direction detection unit
- 131: Second positional deviation detection light source
- 132: Light receiving sensor
- 20: Control unit
- 21: Image information acquisition unit
- 22: Storage unit
- 23: Positional deviation amount calculation unit
- 24: Position correction unit
- 25: Image information acquisition unit
- 26: Image processing unit
- 27: Adjustment information storage unit
- 30: Drive mechanism unit
- 31: X-direction drive mechanism
- 32: Y-direction drive mechanism
- 33: Z-direction drive mechanism
- 40: Monitor

## Claims

1. A tear film photographing apparatus that captures images of interference fringes formed by a tear film of an eye to be examined, the tear film photographing apparatus comprising:
a first optical system including an objective lens disposed to face the eye to be examined, a first illumination unit that irradiates the eye to be examined with first light through the objective lens, and a first image capturing unit that receives the first light, which has been reflected by the tear film of the eye to be examined and has passed through the objective lens, and captures the images of the interference fringes formed by the tear film;
a second optical system including a second illumination unit that irradiates the eye to be examined with second light, a second light reflecting unit that is disposed on an optical path of the first optical system and selectively reflects the second light reflected by a corneal surface of the eye to be examined and passing through the objective lens, and a second image capturing unit that receives the second light via the second light reflecting unit and captures an image of an anterior eye portion of the eye to be examined, the second optical system including optical components whose positional relationship is adjusted in such a manner that an image capturing range of the second image capturing unit is wider than an image capturing range of the first image capturing unit; and
an image processing unit that generates a composite image of an interference fringe image captured by the first optical system and an anterior eye portion image captured by the second optical system.

2. The tear film photographing apparatus according to claim 1, wherein
the second optical system includes a filter unit configured to allow insertion and removal of an infrared light transmitting filter on an optical path between the second light reflecting unit and the second image capturing unit,
the second illumination unit irradiates the eye to be examined with the second light including infrared light,
the second light reflecting unit is a dichroic mirror that reflects the infrared light, and
the second optical system inserts the infrared light transmitting filter on the optical path by the filter unit and captures the image of the anterior eye portion using the infrared light by the second image capturing unit.

3. The tear film photographing apparatus according to claim 1, wherein
the second optical system includes a filter unit configured to allow insertion and removal of a visible light transmitting filter on an optical path between the second light reflecting unit and the second image capturing unit,
the second illumination unit irradiates the eye to be examined with the second light including visible light,
the second light reflecting unit is a dichroic mirror that reflects the visible light, and
the second optical system inserts the visible light transmitting filter on the optical path by the filter unit and captures the image of the anterior eye portion using the visible light by the second image capturing unit.

4. The tear film photographing apparatus according to claim 3, wherein
the dichroic mirror has a reflectance with respect to the visible light lower than a reflectance with respect to the infrared light.

5. The tear film photographing apparatus according to claim 1, wherein
the second optical system includes a filter unit configured to allow insertion and removal of a fluorescence transmitting filter on an optical path between the second light reflecting unit and the second image capturing unit,
the second illumination unit irradiates the eye to be examined with the second light including excitation light,
the second light reflecting unit is a dichroic mirror that reflects fluorescence, and
the second optical system inserts the fluorescence transmitting filter on the optical path by the filter unit and captures the image of the anterior eye portion using the fluorescence by the second image capturing unit.

6. The tear film photographing apparatus according to claim 5, wherein
the image processing unit measures a non-invasive tear break-up time using the interference fringe image obtained by the first optical system, and measures a fluorescein tear break-up time using the anterior eye portion image obtained by the second optical system.

7. The tear film photographing apparatus according to any one of claims 1 to 6, wherein
the second optical system is capable of switching between an image capturing operation of capturing the image of the anterior eye portion of the eye to be examined and an alignment operation of detecting a position of the first optical system relative to the eye to be examined, and performs binning during the alignment operation as compared with the image capturing operation.

8. The tear film photographing apparatus according to claim 1, wherein
the second optical system includes a filter unit on an optical path between the second light reflecting unit and the second image capturing unit,
the second illumination unit is configured to be capable of switching a plurality of beams of light as the second light,
the filter unit is capable of selectively switching a plurality of optical filters to transmit each of the beams of light that is the second light, and
the second optical system switches irradiation light of the second illumination unit and each of the optical filters of the filter unit in conjunction with one another.

9. The tear film photographing apparatus according to any one of claims 1 to 6, wherein
the image processing unit has a function of generating a composite image of the interference fringe image captured by the first optical system and an externally input anterior eye portion image input from an outside, and compares the anterior eye portion image captured by the second image capturing unit with the externally input anterior eye portion image to determine an enlargement/reduction ratio of an image when the interference fringe image is combined with the externally input anterior eye portion image.
